# EUROPEAN PATENT SPECIFICATION

(11) **EP 2 520 212 B1**
(45) Date of publication and mention of the grant of the patent: **13.05.2015**
(21) Application number: 11742111.5
(22) Date of filing: 26.01.2011
(51) Int. Cl.: A61B 1/00, A61B 1/04, G01N 21/64, H04N 7/18

(54) **FLUORESCENCE ENDOSCOPE DEVICE**
FLUORESZENZENDOSKOPVORRICHTUNG
DISPOSITIF D'ENDOSCOPE À FLUORESCENCE

(30) Priority: 10.02.2010 JP 2010027885
(43) Date of publication of application: 07.11.2012
(73) Proprietor: Olympus Corporation, Shibuya-ku Tokyo 151-0072 (JP)
(72) Inventor: ISHIHARA, Yasushige, Tokyo 151-0072 (JP)
(74) Representative: Schicker, Silvia
(86) International application number: PCT/JP2011/051453
(87) International publication number: WO 2011/099364

(56) References cited:
- EP-A1- 2 108 300
- JP-A- 2001 169 999
- JP-A- 2002 263 064
- JP-A- 2003 079 568
- JP-A- 2009 225 851

## Description

### {Technical Field}

The present invention relates to fluorescence endoscope devices.

### {Background Art}

A known fluorescence endoscope device in the related art can obtain a bright fluorescence image of a diseased site by irradiating an observation target site doped with a fluorochrome that preferentially accumulates in a diseased site, such as a cancer cell, with excitation light for exciting the fluorochrome to generate drug fluorescence and by capturing an image of the drug fluorescence (for example, see PTL 1). The fluorescence endoscope device disclosed in PTL 1 corrects variations in fluorescence intensity of a fluorescence image, which depends on the observation distance, the observation angle, etc., by dividing a fluorescence image, which is obtained from an observation target site irradiated with excitation light and is based on the fluorescence intensity, by a reference image, which is obtained from the same observation target site irradiated with reference light and is based on the intensity of the reflected light.
Document EP 2 108 300 A1 relates to a fluorescence observation apparatus. The fluorescence observation apparatus is provided with a light source unit, an insertion unit, an image processing unit and a monitor. The light source unit includes a white light source for generating white light. An objective lens collects reflected light or fluorescence returning from an observation target site S.
A reflected-light image-acquisition device detects the reflected light and a fluorescence-image-acquisition device detects the fluorescence. Then, a reflected-light image generating unit generates a reflected-light image based on information sent from the reflected-light image-acquisition unit and a fluorescence-image generating unit generates an autofluorescence image and an agent-fluorescence image based on information sent from the fluorescence-image-acquisition unit. Thereafter, a blood-vessel image-extraction unit extracts a blood-vessel image from the reflected-light image, a reference-image generating unit generates a reference image based on the autofluorescence image and the blood-vessel image, and a fluorescence-image correcting unit corrects the agent-fluorescence image based on the generated reference image.

### {Citation List}

### {Patent Literature}

{PTL 1} Japanese Unexamined Patent Application, Publication No. 2006-175052

### {Summary of Invention}

### {Technical Problem}

However, because fluorescence and reflected light have different characteristics in terms of their dependence on the observation distance and the observation angle, the influence of observation distance and observation angle cannot be completely corrected by dividing a fluorescence image by a reference image, as in the fluorescence endoscope device disclosed in PTL 1. For example, there is a problem in that a variation occurs between the center and periphery of an image, because the excitation light and the reference light have different irradiation distributions depending on the observation distance and the observation angle.

The present invention has been made in view of these circumstances, and an object thereof is to provide a fluorescence endoscope device that can obtain quantitative information of a subject.

### {Solution to Problem}

To overcome the above-described problems, the present invention employs the following solutions.

An aspect of the present invention is a fluorescence endoscope device including a light source that irradiates a subject with excitation light and reference light; a fluorescence-image acquisition portion that captures an image of fluorescence generated at the subject due to the irradiation with the excitation light from the light source to obtain a fluorescence image; a reference-image acquisition portion that captures an image of return light returning from the subject due to the irradiation with the reference light from the light source to obtain a reference image; a corrected-fluorescence-image generating portion that corrects the fluorescence image obtained by the fluorescence-image acquisition portion using the reference image obtained by the reference-image acquisition portion to generate a corrected fluorescence image; an effective-area defining portion that defines an effective area having a brightness in a predetermined variation range in the corrected fluorescence image generated by the corrected-fluorescence-image generating portion; a region-extracting portion that extracts a high-brightness region having a brightness higher than or equal to a predetermined threshold in the corrected fluorescence image; and an indication portion that shows whether or not the high-brightness region extracted by the region-extracting portion exists inside the effective area in an identifiable manner.

In the present invention, when the subject is irradiated with the excitation light emitted from the light source, the fluorescence-image acquisition portion acquires a fluorescence image of fluorescence generated at the subject. Furthermore, when the subject is irradiated with the reference light emitted together with the excitation light from the light source, the reference-image acquisition portion acquires a reference image of the return light. Then, the corrected-fluorescence-image generating portion corrects the fluorescence image of the subject using the reference image of the same subject, whereby a corrected fluorescence image is generated, in which the variation in fluorescence intensity, which depends on the observation distance and the observation angle, is reduced.

Because the excitation light and the reference light have different irradiation distributions depending on the observation distance and the observation angle, the brightness of the corrected fluorescence image may vary depending on the position in the image. In this case, as a result of the effective-area defining portion defining an effective area in the corrected fluorescence image, the high-brightness region extracted by the region-extracting portion can be classified into one having a high effective brightness and one having a low effective brightness. Thus, as a result of the indication portion showing whether or not the high-brightness region exists inside the effective area in an identifiable manner, effective and quantitative information of the subject having little variation due to the influence of observation distance and observation angle can be selectively obtained.

In the above-described aspect, the corrected-fluorescence-image generating portion may divide the fluorescence image by the reference image.

With this configuration, a highly quantitative corrected fluorescence image can be generated by simple calculation.

In the above-described aspect, the indication portion may show the effective area on the corrected fluorescence image such that the effective area can be distinguished from the other area.

With this configuration, the high-brightness region existing inside the effective area in the corrected fluorescence image can be easily identified on the corrected fluorescence image.

In the above-described aspect, the indication portion may show image information of the high-brightness region.

With this configuration, the detailed information about the high-brightness region existing inside the effective area in the corrected fluorescence image can be easily obtained. Examples of the image information include the average brightness and the area of the high-brightness region.

In the above-described aspect, the indication portion may show the image information of the high-brightness region in a vicinity thereof on the corrected fluorescence image.

With this configuration, the image information of the effective high-brightness region can be easily obtained on the corrected fluorescence image.

In the above-described aspect, the effective-area defining portion may define a predetermined area from a center of the corrected fluorescence image as the effective area.

The smaller the observation distance and the observation angle with respect to the subject are, the smaller the variation in brightness tends to be, and the larger the observation distance and the observation angle are, the larger the variation in brightness tends to be. With this configuration, a reliable effective area can be defined when the subject is observed perpendicularly.

In the above-described aspect, the effective-area defining portion may define an effective area measured by using a standard sample that generates fluorescence of uniform intensity over an observation area as the subject as an effective area in a corrected fluorescence image of another subject.

The variation in brightness due to the influence of observation distance and observation angle clearly appears in the corrected fluorescence image of the standard sample that generates fluorescence of uniform intensity over the observation area. Accordingly, it is possible to define a precise effective area in the corrected fluorescence image of another subject, using the measured brightness in the corrected fluorescence image of the standard sample.

In the above-described aspect, an endoscope scope including, at a tip thereof, a light-emitting portion that emits the excitation light and the reference light and a light-receiving portion that receives the fluorescence and the return light may be provided, and the effective-area defining portion may define an effective area on the basis of scope information about the light-emitting portion and the light-receiving portion of the endoscope scope.

With this configuration, a practical and reliable effective area can be defined with respect to endoscope scopes each having different uses and specifications according to the observation object and the observation method. Examples of the scope information include the number of light-emitting portions and the observation angles of the light-emitting portion and light-receiving portion. For example, when light is emitted from a single light-emitting portion, a circular effective area centered on the optical axis thereof may be defined, and when light is emitted from a plurality of light-emitting portions, an elliptical effective area containing all the irradiation areas irradiated with the light-emitting portions may be defined. Furthermore, when the subject is observed perpendicularly, a predetermined area from the center of the image may be defined as the effective area, and when the wall of the body cavity in a lumen is observed at a wide angle, a ring-like effective area excluding the central portion of the image may be defined.

### {Advantageous Effects of Invention}

The present invention provides an advantage in that quantitative information of a subject can be obtained.

### {Brief Description of Drawings}

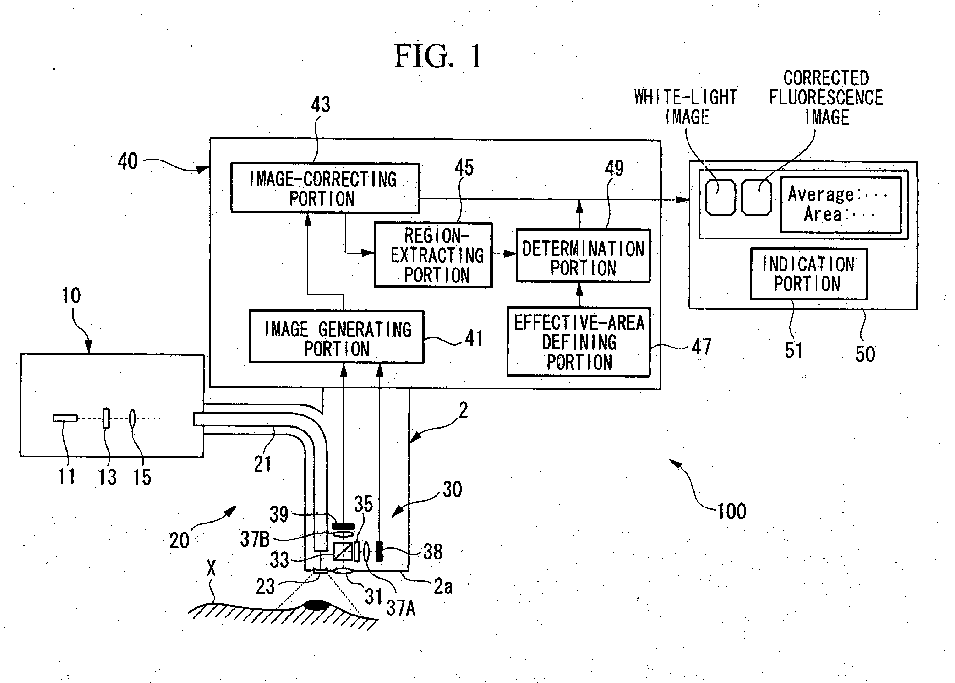

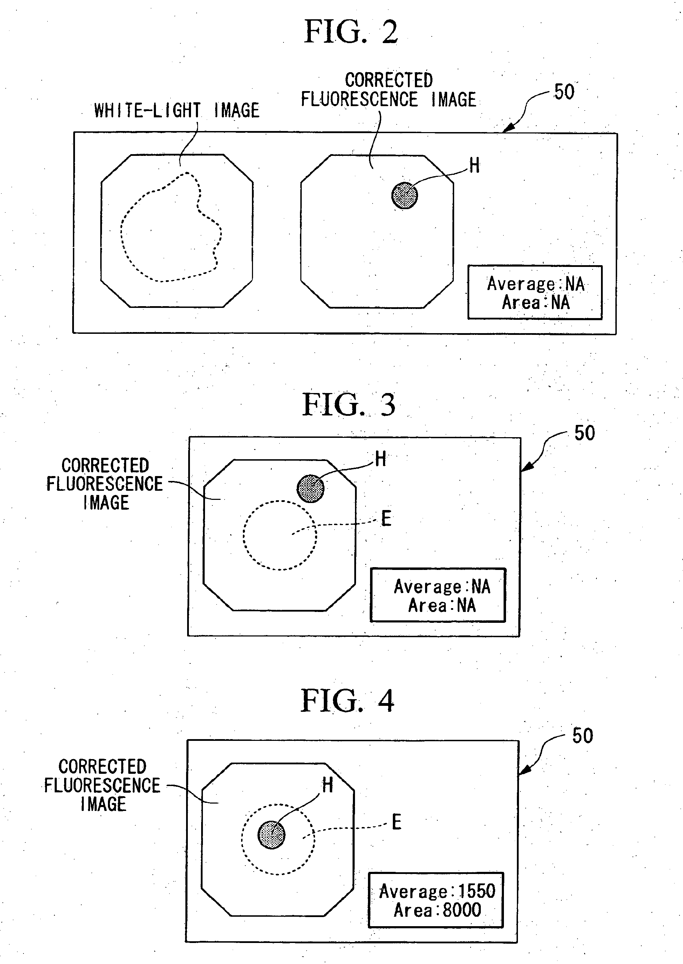

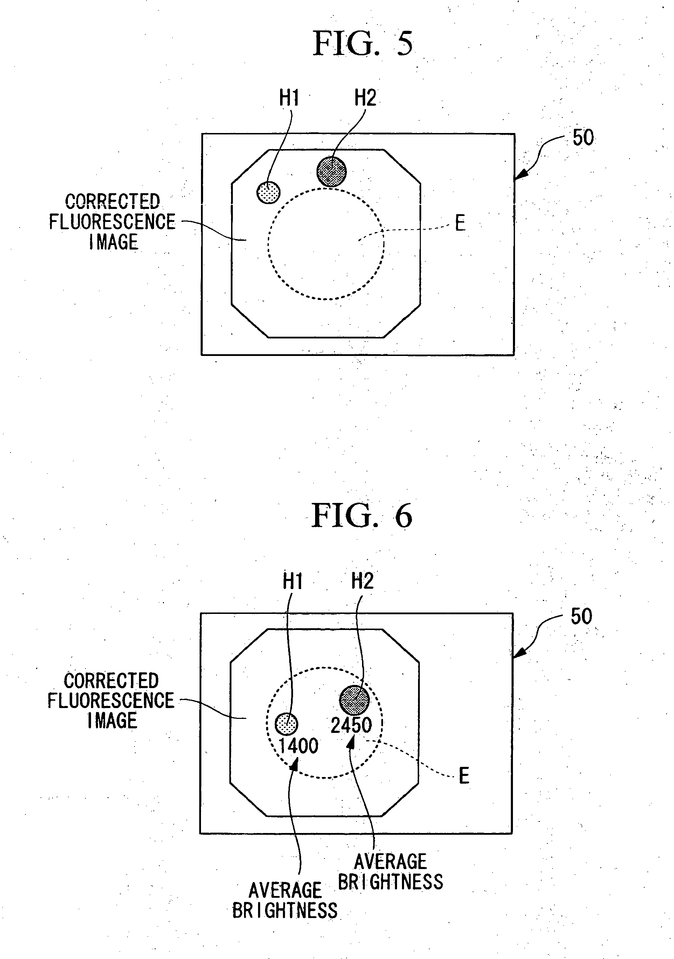

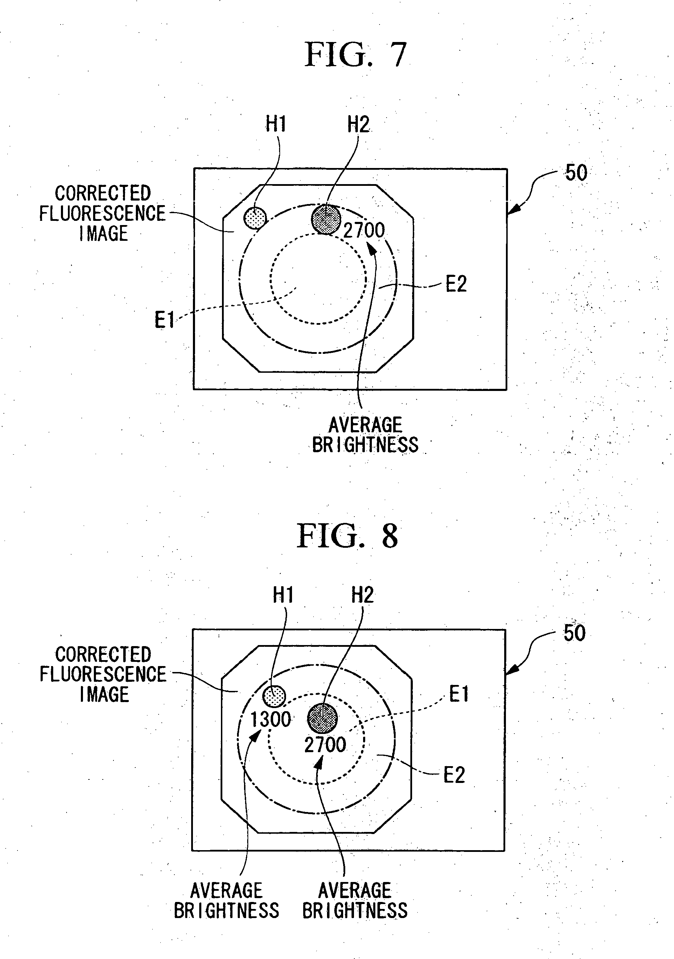

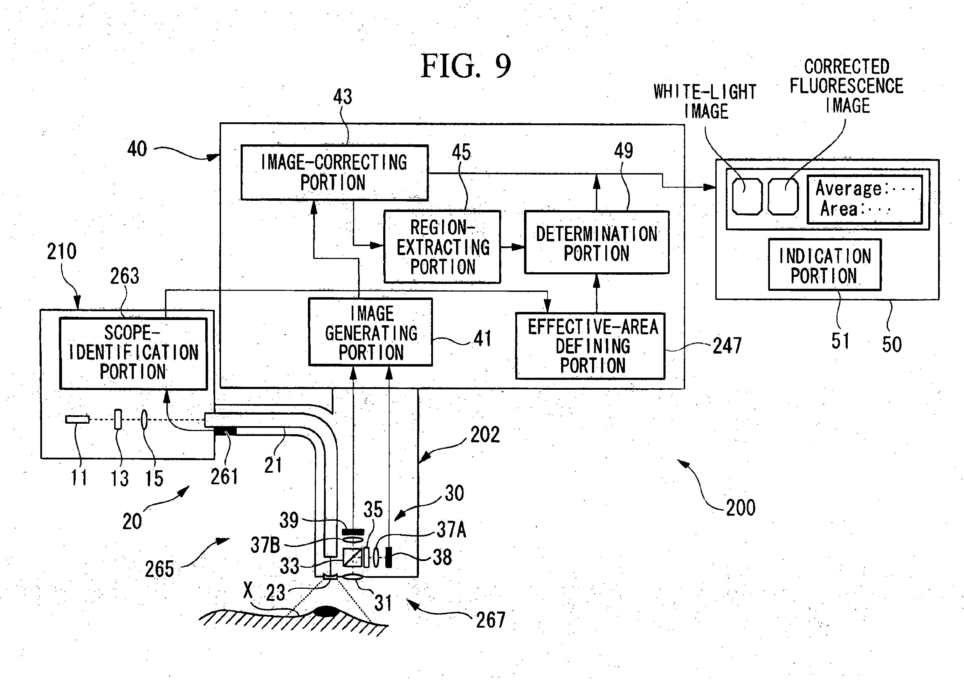

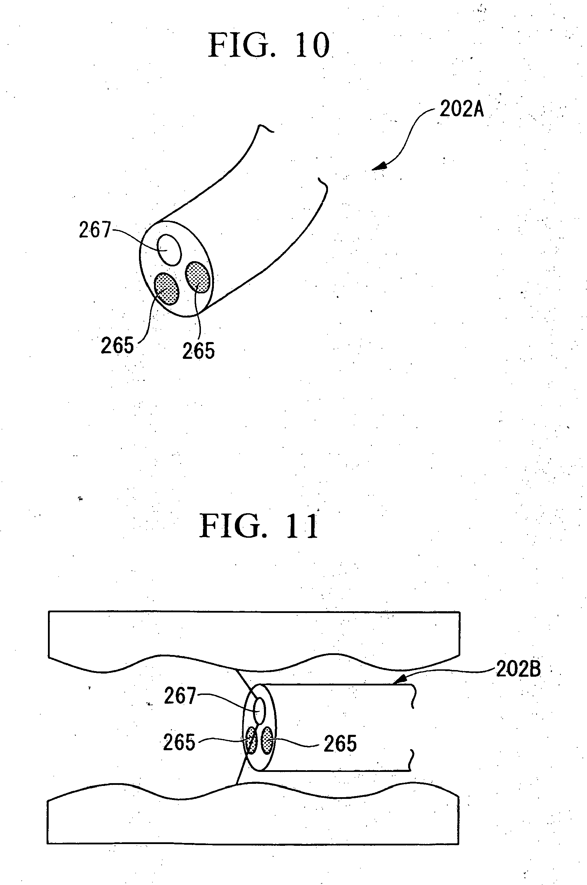

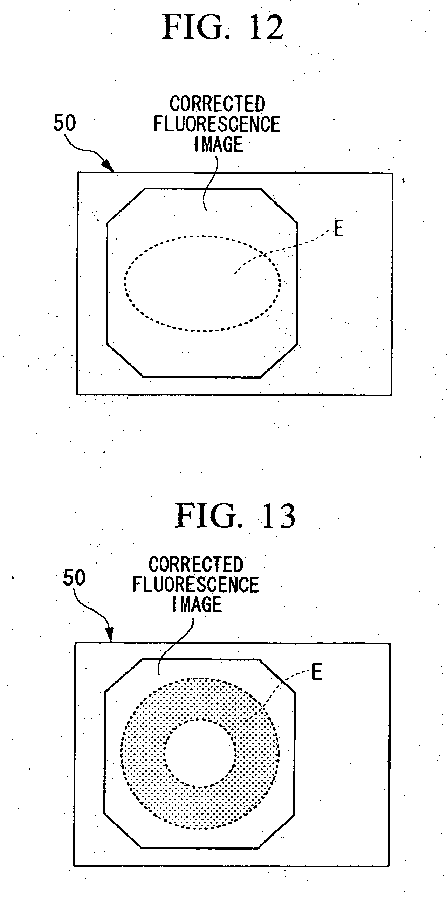

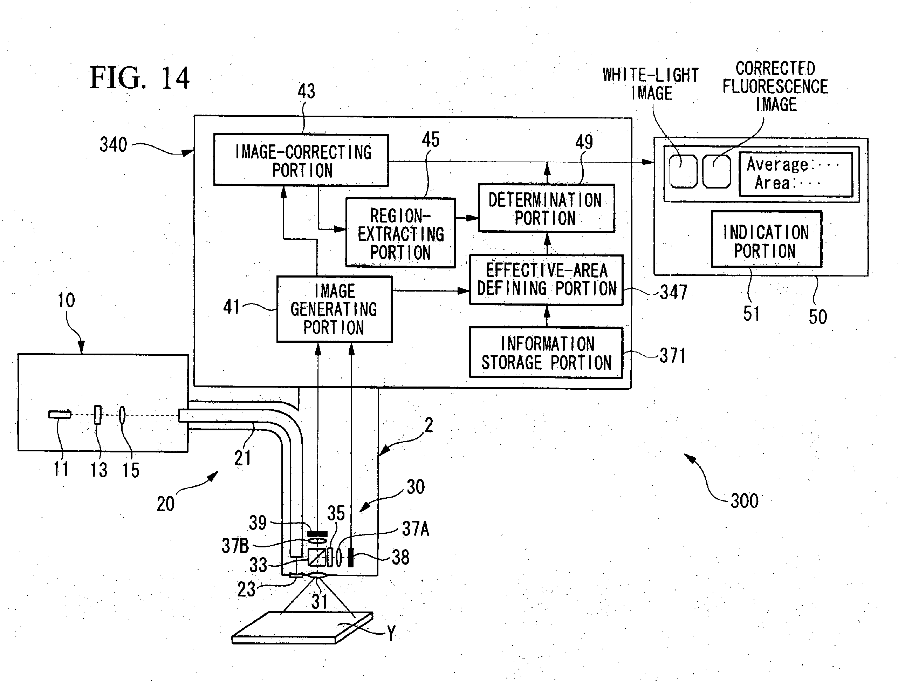

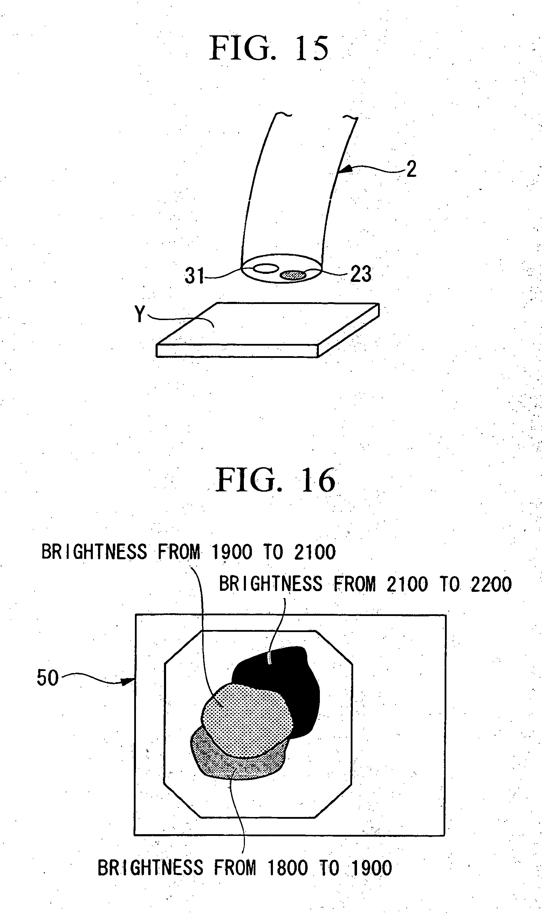

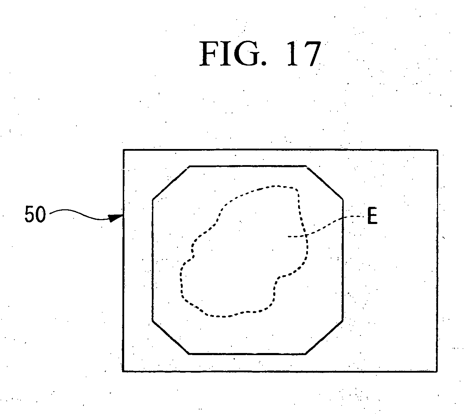

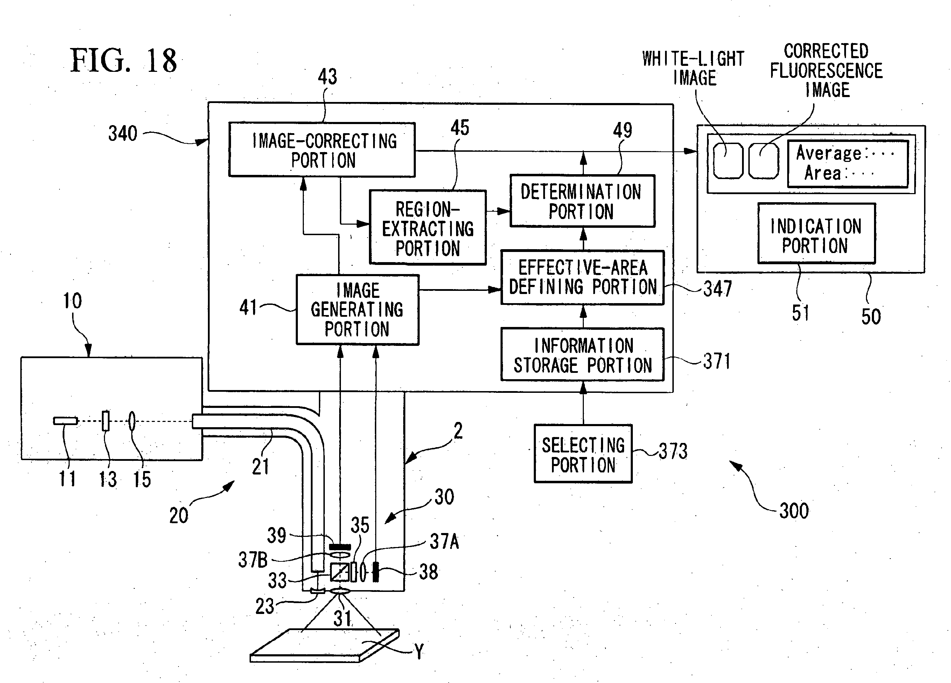

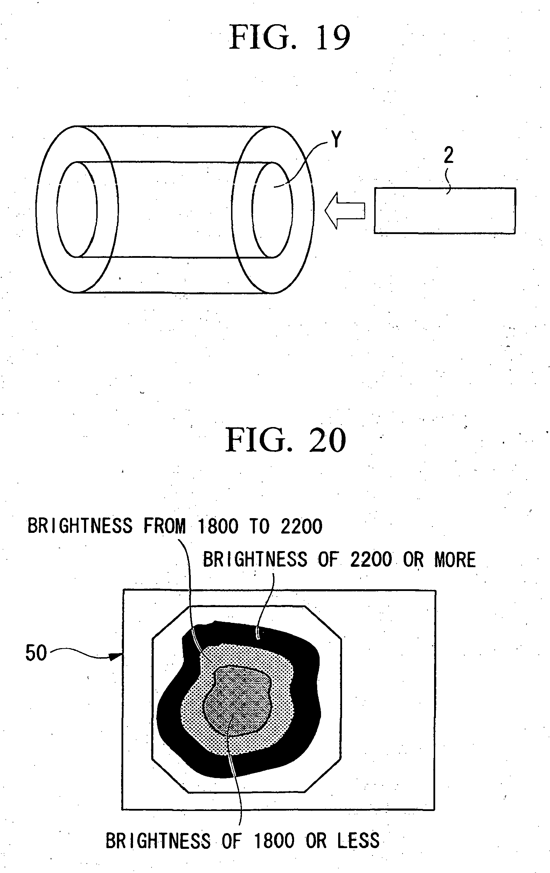

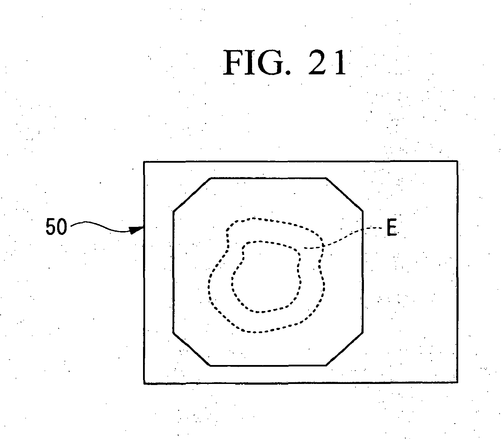

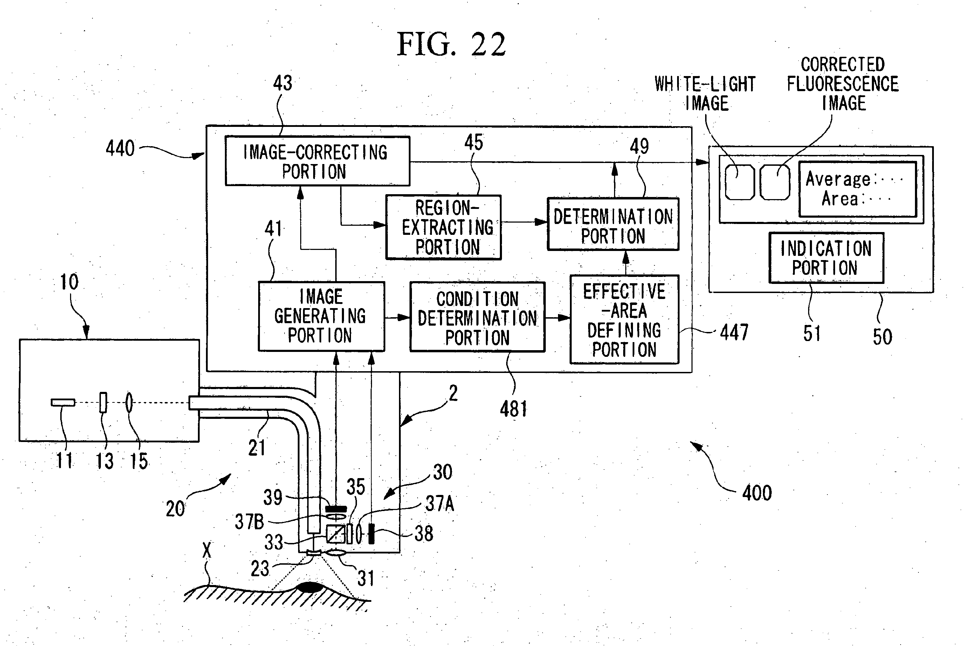

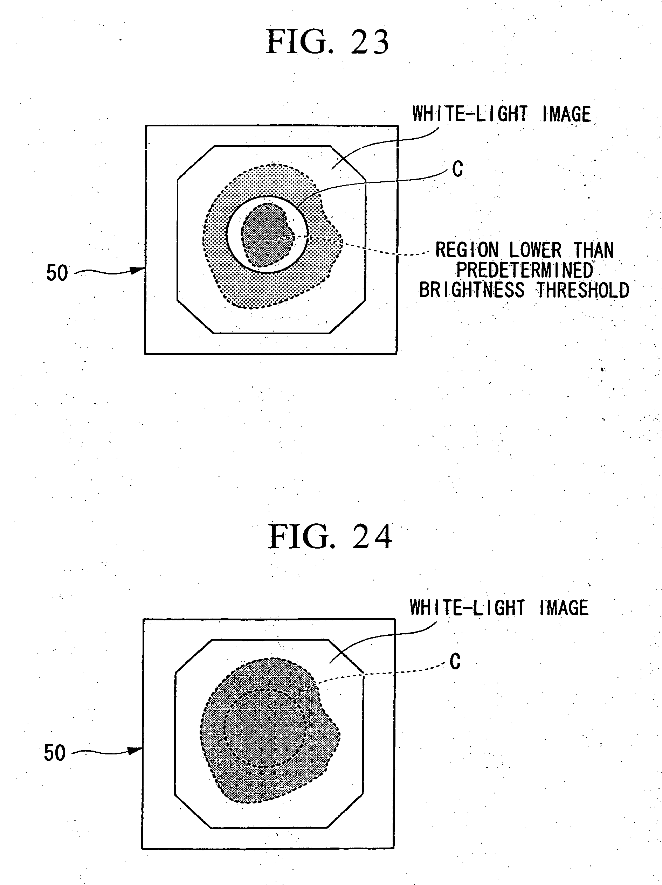

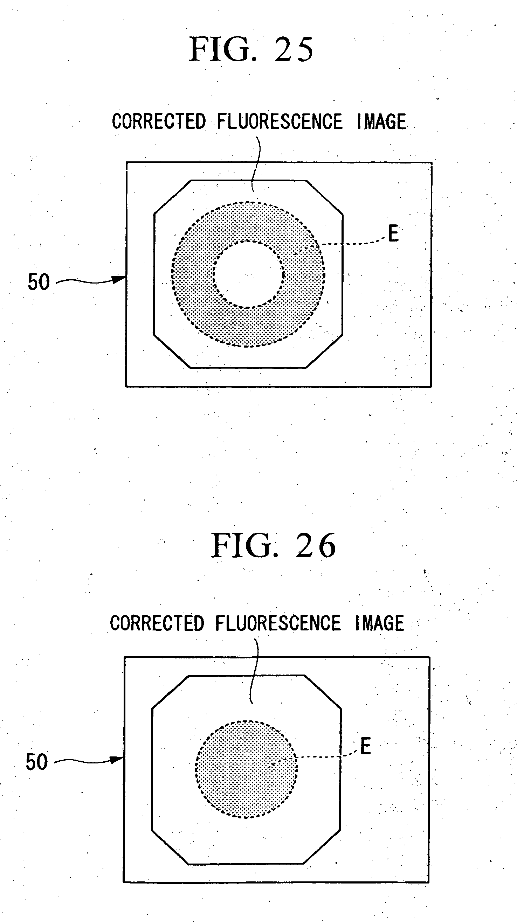

{FIG. 1} FIG. 1 is a schematic block diagram of a fluorescence endoscope device according to a first embodiment of the present invention.
{FIG. 2} FIG. 2 is an enlarged view of a monitor in FIG. 1.
{FIG. 3} FIG. 3 is a diagram showing a case where an extracted high-brightness region exists outside an effective area.
{FIG. 4} FIG. 4 is a diagram showing a case where the extracted high-brightness region exists inside the effective area.
{FIG. 5} FIG. 5 is a diagram showing a case where a plurality of high-brightness regions, extracted by a fluorescence endoscope device according to a first modification of the first embodiment of the present invention, exist outside an effective area.
{FIG. 6} FIG. 6 is a diagram showing a case where a plurality of high-brightness regions, extracted by the fluorescence endoscope device according to the first modification of the first embodiment of the present invention, exist inside the effective area.
{FIG. 7} FIG. 7 is a diagram showing a case where one of a plurality of high-brightness regions, extracted by a fluorescence endoscope device according to a second modification of the first embodiment of the present invention, exists in one of a plurality of effective areas.
{FIG. 8} FIG. 8 is a diagram showing a case where each of the plurality of high-brightness regions, extracted by the fluorescence endoscope device according to the second medification of the first embodiment of the present invention, exists in one of the plurality of effective areas.
{FIG. 9} FIG. 9 is a schematic block diagram of a fluorescence endoscope device according to a second embodiment of the present invention.
{FIG. 10} FIG. 10 is a schematic diagram showing another scope that may be interchanged with a scope in FIG. 9.
{FIG. 11} FIG. 11 is a schematic diagram showing another scope that may be interchanged with the scope in FIG. 9.
{FIG. 12} FIG. 12 is a corrected fluorescence image obtained by using the scope in FIG. 10.
{FIG. 13} FIG. 13 is a corrected fluorescence image obtained by using the scope in FIG. 11.
{FIG. 14} FIG. 14 is a schematic block diagram of a fluorescence endoscope device according to a third embodiment of the present invention.
{FIG. 15} FIG. 15 is an enlarged view of the scope in FIG. 13 and a phantom.
{FIG. 16} FIG. 16 is a diagram showing the brightness distribution in a corrected fluorescence image of the phantom in FIG. 15.
{FIG. 17} FIG. 17 is a diagram showing an effective area of the phantom in FIG. 15.
{FIG. 18} FIG. 18 is a schematic block diagram of a fluorescence endoscope device according to a modification of the third embodiment of the present invention.
{FIG. 19} FIG. 19 is an enlarged view of another phantom and scope.
{FIG. 20} FIG. 20 is a diagram showing a corrected fluorescence image of the phantom in FIG. 19.
{FIG. 21} FIG. 21 is a diagram showing an effective area of the phantom in FIG. 19.
{FIG. 22} FIG. 22 is a schematic block diagram of a fluorescence endoscope device according to a modification of a fourth embodiment of the present invention.
{FIG. 23} FIG. 23 is a diagram showing a white-light image generated by an image generating portion in FIG. 22.
{FIG. 24} FIG. 24 is a diagram showing another white-light image generated by the image generating portion in FIG. 22.
{FIG. 25} FIG. 25 is a diagram showing a corrected fluorescence image that uses the white-light image in FIG. 23.
{FIG. 26} FIG. 26 is a diagram showing a corrected fluorescence image that uses the white-light image in FIG. 24.

### {Description of Embodiments}

### First Embodiment

A fluorescence endoscope device according to a first embodiment of the present invention will be described below with reference to the drawings.

As shown in FIG. 1, a fluorescence endoscope device 100 according to this embodiment includes a long, thin scope 2 to be inserted into a body cavity, an illuminating unit 20 including a light source 10 that emits illumination light from a tip 2a of the scope 2, an image-capturing unit 30 disposed in the scope 2 to obtain image information of an observation target site X, i.e., a subject, an image-processing section 40 that processes the image information obtained by the image-capturing unit 30, and a monitor 50 on which the image and image information processed by the image-processing section 40 are displayed.

The light source 10 includes a xenon lamp (Xe lamp) 11 that emits illumination light, an excitation light filter 13 that separates white light (reference light) containing excitation light from the illumination light emitted from the xenon lamp 11, and a coupling lens 15 that collects the white light containing the excitation light, separated by the excitation light filter 13. The excitation light filter 13 separates white light containing excitation light with a wavelength band of, for example, from 400 nm to 740 nm.

Furthermore, the illuminating unit 20 includes a light-guide fiber 21 disposed substantially over the overall length of the scope 2 in the longitudinal direction, and a spreading lens 23 disposed at the tip 2a of the scope 2.

The light-guide fiber 21 guides the white light containing the excitation light, collected by the coupling lens 15, to the tip 2a of the scope 2. The spreading lens 23 spreads the white light containing the excitation light, guided by the light-guide fiber 21, to illuminate the observation target site X.

The image-capturing unit 30 includes an objective lens 31 that collects return light returning from the observation target site X irradiated with the white light containing the excitation light by the illuminating unit 20, and a beam splitter 33 that splits the return light, collected by the objective lens 31, according to the wavelength.

The objective lens 31 is disposed beside the spreading lens 23 at the tip 2a of the scope 2. In the return light, the beam splitter 33 reflects light (excitation light and fluorescence) having a longer wavelength than the excitation wavelength and allows white light (return light) that has a shorter wavelength than the excitation wavelength to pass therethrough.

This image-capturing unit 30 includes an excitation-light cut filter 35 that, of the excitation light and fluorescence reflected by the beam splitter 33, blocks excitation light and allows only fluorescence (for example, near-infrared fluorescence) to pass therethrough, a focusing lens 37A that focuses fluorescence passing through the excitation-light cut filter 35, a focusing lens 37B that focuses white light passing through the beam splitter 33, a fluorescence-image-capturing portion 38 that captures an image of the fluorescence focused by the focusing lens 37A, and a white-light image-capturing portion 39 that captures an image of the white light focused by the focusing lens 37B.

For example, the excitation-light cut filter 35 allows only fluorescence in the wavelength band from 765 nm to 850 nm to pass therethrough. The fluorescence-image-capturing portion 38 is, for example, a highly sensitive monochrome CCD for fluorescence. This fluorescence-image-capturing portion 38 obtains fluorescence image information by capturing an image of fluorescence. The white-light image-capturing portion 39 is, for example, a color CCD for white light and has a mosaic filter (not shown). This white-light image-capturing portion 39 obtains white-light image information by capturing an image of white light.

The image-processing section 40 includes an image generating portion (a fluorescence-image acquisition portion and a reference-image acquisition portion) 41 that generates a fluorescence image and a white-light image (reference image), and an image-correcting portion (corrected-fluorescence-image generating portion) 43 that corrects the fluorescence image generated by the image generating portion 41 with the white-light image.

The image generating portion 41 generates a two-dimensional fluorescence image from the fluorescence image information obtained by the fluorescence-image-capturing portion 38 and generates a two-dimensional white-light image from the white-light image information obtained by the white-light image-capturing portion 39.

The image-correcting portion 43 corrects the fluorescence image by dividing the fluorescence image of the observation target site X by the white-light image of the same observation target site X. By doing so, a corrected fluorescence image is generated, in which the variation in fluorescence intensity of the fluorescence image, which depends on the observation distance, the observation angle, etc., is reduced. Furthermore, the image-correcting portion 43 outputs the white-light image and the generated corrected fluorescence image to the monitor 50.

Furthermore, the image-processing section 40 includes a region-extracting portion 45 that extracts a region of pixels having a higher brightness than a predetermined threshold (hereinbelow, a "high-brightness region") in the corrected fluorescence image generated by the image-correcting portion 43, an effective-area defining portion 47 that defines an area in which the brightness is effective (hereinbelow, "effective area") in the corrected fluorescence image, and a determination portion 49 that determines whether or not the high-brightness region extracted by the region-extracting portion 45 exists inside the effective area.

The predetermined threshold is inputted to the region-extracting portion 45 in advance.

The effective-area defining portion 47 defines, as an effective area, a region of pixels that are indicated with a fluorescence intensity in a predetermined variation range (for example, ±10% or ±20%) with respect to the fluorescence intensity of pixels at the center of the corrected fluorescence image.

For example, the inside of a circle having a predetermined radius from the center of the image (a predetermined number of pixels) is defined as the effective area. The smaller the observation distance and the observation angle with respect to the subject are, the smaller the variation in brightness tends to be, and the larger the observation distance and the observation angle with respect to the subject are, the larger the variation in brightness tends to be; therefore, a reliable effective area can be defined when the subject is observed perpendicularly.

The determination portion 49 calculates image information of the high-brightness region that is determined to exist inside the effective area and does not calculate image information of the high-brightness region that is determined not to exist inside the effective area. Examples of the image information include the average brightness and the area of the high-brightness region.

The monitor 50 can simultaneously display the white-light image and the corrected fluorescence image sent from the image-correcting portion 43. Furthermore, the monitor 50 includes an indication portion 51 that shows the outline of the effective area defined by the effective-area defining portion 47 on the corrected fluorescence image and shows the image information of the high-brightness region calculated by the determination portion 49.

When the determination portion 49 determines that the high-brightness region is contained in the effective area, the indication portion 51 shows the calculated image information, more specifically, the average brightness (Average) and the area (Area) of the high-brightness region. Furthermore, when the determination portion 49 determines that the high-brightness region is not contained in the effective area, the indication portion 51 shows NA (Not Available) in the fields of the average brightness (Average) and area (Area) of the high-brightness region.

The operation of the thus-configured fluorescence endoscope device 100 according to this embodiment will now be described.

When an observation target site X inside the body cavity of a living body is observed perpendicularly using the fluorescence endoscope device 100 according to this embodiment, a fluorochrome is attached to or caused to be absorbed in the observation target site X. Then, the light source 10 is activated, and the tip 2a of the scope 2 is inserted into the body cavity and is made to face the observation target site X.

The white light containing excitation light, which is emitted from the xenon lamp 11 and is separated by the excitation light filter 13, is collected by the coupling lens 15 and is guided to the tip 2a of the scope 2 by the light-guide fiber 21. The white light containing the excitation light is then spread by the spreading lens 23 and illuminates the observation target site X.

At the observation target site X, a fluorescent substance contained therein is excited by the excitation light and emits fluorescence, and portions of the white light and the excitation light are reflected at the surface. The fluorescence, the white light, and the excitation light are collected by the objective lens 31, and the beam splitter 33 reflects light having a longer wavelength than the excitation wavelength, i.e., excitation light and fluorescence, and allows the white light that has a shorter wavelength than the excitation wavelength to pass therethrough.

The excitation light and fluorescence reflected by the beam splitter 33 are incident on the excitation-light cut filter 35, where the excitation light is removed. Then, only the fluorescence is focused by the focusing lens 37A, and the fluorescence-image-capturing portion 38 captures the image thereof. Thus, the fluorescence-image-capturing portion 38 obtains the fluorescence image information of the observation target site X. The white light passing through the beam splitter 33 is focused by the focusing lens 37B, and the image thereof is captured by the white-light image-capturing portion 39. Thus, the white-light image-capturing portion 39 obtains the white-light image information of the observation target site X. Either of the fluorescence image information and the white-light image information may be obtained first; or they may be obtained simultaneously.

The fluorescence image information obtained by the fluorescence-image-capturing portion 38 and the white-light image information obtained by the white-light image-capturing portion 39 are inputted to the image generating portion 41 of the image-processing section 40. The image generating portion 41 generates a two-dimensional fluorescence image on the basis of the fluorescence image information and generates a two-dimensional white-light image on the basis of the white-light image information.

The fluorescence image and white-light image generated by the image generating portion 41 are sent to the image-correcting portion 43. In the image-correcting portion 43, a corrected fluorescence image is generated by dividing the fluorescence image by the white-light image. The generated corrected fluorescence image is sent to the region-extracting portion 45 and is then sent to the monitor 50, along with the white-light image, to be displayed, as shown in FIG. 2.

In the region-extracting portion 45, as shown in the figure mentioned above, a group of pixels in the corrected fluorescence image having a higher brightness than a predetermined threshold inputted in advance are extracted as a high-brightness region H.

For example, when fluorescence within a range of variation of +10% with respect to fluorescence having an intensity indicated as a brightness of 2000 at the center of the corrected fluorescence image is to be observed, the effective-area defining portion 47 defines a circular region in which the fluorescence intensity is indicated as a brightness from 1800 to 2200 as an effective area E. Then, as shown in FIGS. 3 and 4, the indication portion 51 shows the outline of the defined effective area E on the corrected fluorescence image.

In the determination portion 49, when an extracted high-brightness region H exists outside the effective area E, as shown in FIG. 3, the determination portion 49 determines that the high-brightness region H is not contained in the effective area E. Furthermore, the indication portion 51 shows "NA" in the fields "Average" and "Area".

On the other hand, when the extracted high-brightness region H exists inside the effective area E, as shown in FIG. 4, the determination portion 49 determines that the high-brightness region H is contained in the effective area E, and image information thereof is calculated. Furthermore, the indication portion 51 shows the calculated values in the fields "Average" and "Area" of the high-brightness region H. In this figure, "Average" of the high-brightness region H is 1550 and "Area" of the high-brightness region H is 8000.

As has been described above, with the fluorescence endoscope device 100 according to this embodiment, even when a variation in brightness occurs in the corrected fluorescence image depending on the position in the image due to the difference of irradiation distribution between excitation light and reference light, by calculating and displaying image information of only the high-brightness region H existing inside the effective area E, effective and quantitative image information having little variation due to the influence of observation distance and observation angle can be selectively obtained.

Although the image information of only the high-brightness region H existing inside the effective area E is calculated and displayed in this embodiment, it is only necessary to show whether or not the high-brightness region H exists inside the effective area E in an identifiable manner. For example, image information of the high-brightness region existing outside the effective area E may also be displayed, as long as the inside of the effective area E can be distinguished from the outside of the effective area E by, for example, showing the outline of the effective area E or by differentiating the color of the inside of the effective area E from the color of the other region. Furthermore, it is also possible not to show the effective area E on the corrected fluorescence image, by not showing the high-brightness region outside the effective area E on the image or by not showing the image information thereof, and by showing only the high-brightness region H existing inside the effective area E on the image or by showing the image information thereof.

This embodiment may be modified as follows.

For example, although the region-extracting portion 45 extracts one high-brightness region H in the corrected fluorescence image in this embodiment, in a first modification, two or more high-brightness regions H in the same corrected fluorescence image may be simultaneously extracted. In this case, the determination portion 49 may calculate only the image information of the high-brightness region H existing inside the effective area E and not the image information of the high-brightness region H that does not exist inside the effective area E. Furthermore, the indication portion 51 may show the calculated image information near the high-brightness region H on the image.

For example, if neither extracted high-brightness region H1 nor H2 exists inside the effective area E, as shown in FIG. 5, the image information is not calculated for either of the high-brightness regions H1 and H2. In contrast, if extracted high-brightness regions H1 and H2 exist inside the effective area E, as shown in FIG. 6, the image information of both of the high-brightness regions H1 and H2 is calculated and shown near the corresponding regions (in the figure, the average brightness of the high-brightness region H1: 1400, and the average brightness of the high-brightness region H2: 2450). By doing so, the image information of the plurality of high-brightness regions H1 and H2 can be easily ascertained on the corrected fluorescence image.

Furthermore, although one effective area E is defined on the corrected fluorescence image in this embodiment, a plurality of effective areas E may be simultaneously defined in the same corrected fluorescence image in a second modification. In this case, for example, as shown in FIGS. 7 and 8, the effective-area defining portion 47 may define, as an effective area E1, a region in which fluorescence is indicated with a variation of ±10% (a brightness from 1800 to 2000) with respect to fluorescence indicated with a brightness of 2000 at the center of the corrected fluorescence image and may define, as an effective area E2, a region in which fluorescence is indicated with a variation of ±20% (a brightness from 1600 to 2400) with respect to the above fluorescence.

Furthermore, for example, if the extracted high-brightness region H2 exists inside the effective area E2, as shown in FIG. 7, the indication portion 51 shows the image information of the high-brightness region H2 in the vicinity thereof (in the figure, the average brightness of the high-brightness region H2: 2700), and, concerning the high-brightness region H1, which is not contained in either of the effective areas E1 and E2, nothing is shown in the vicinity thereof.

Furthermore, if the high-brightness region H1 exists inside the effective area E2 and the high-brightness region H2 exists inside the effective area E1, as shown in FIG. 8, the image information of the high-brightness regions H1 and H2 may be shown in the vicinity thereof (in the figure, the average brightness of the high-brightness region H1: 1300, and the average brightness of the high-brightness region H2: 2700). In this case, the inner parts of the effective areas E1 and E2 may be shown in different colors, or the image information of the effective areas E1 and E2 may be shown in different colors. By doing so, the image information can be easily ascertained according to the desired degree of variation in brightness.

In this modification, because the brightness of the high-brightness region H1 has a variation range of ±20%, it is understood that the fluorescence intensity thereof has a brightness in the range from 1040 to 1560. Furthermore, because the brightness of the high-brightness region H2 has a variation range of ±10%, it is understood that the fluorescence intensity thereof has a brightness in the range from 2430 to 2970.

### Second Embodiment

Next, a fluorescence endoscope device according to a second embodiment of the present invention will be described.

A fluorescence endoscope device 200 according to this embodiment differs from that according to the first embodiment in that, as shown in FIG. 9, a scope (endoscope scope) 202 includes an IC chip 261 that stores scope information about the scope 202 and in that a light source 210 includes a scope-identification portion 263 that identifies the scope information stored in the IC chip 261.

Hereinbelow, portions having the same configurations as those of the fluorescence endoscope device 100 according to the first embodiment will be denoted by the same reference numerals, and descriptions thereof will be omitted.

The scope 202 includes a light-emitting portion 265 including a light-guide fiber 21 and a spreading lens 23, and a light-receiving portion 267 including an objective lens 31. The observation angles of the light-emitting portion 265 and light-receiving portion 267 of this scope 202 are set such that an observation target site X is observed perpendicularly.

Furthermore, the scope 202 is provided such that it can be replaced with another scope 202A or 202B that is used for another purpose or has different specifications, as shown in FIGS. 10 and 11. As shown in FIG. 10, the scope 202A has two light-emitting portions 265 and a light-receiving portion 267, whose observation angles are set such that the observation target site X is observed perpendicularly. This scope 202A irradiates a substantially elliptical irradiation area centered on the optical axes of the light-emitting portions 265 with excitation light and reference light.

As shown in FIG. 11, the scope 202B is used when a portion on a slightly peripheral side relative to the center of the image is to be observed and includes two light-emitting portions 265 and a light-receiving portion 267, whose observation angles are set such that the wall of the body cavity or the like is observed at a wide angle in a state in which they are disposed along the lumen of the esophagus, large intestine, or the like. This scope 202B emits the excitation light and the reference light at a wide angle from the light-emitting portion 265 toward the entirety in the circumferential direction, such as the wall of the body cavity or the like.

The IC chip 261 stores specific information of each of the scopes 202, 202A, and 202B and is disposed at the base end of each of the scopes 202, 202A, and 202B on the light source side. Examples of the scope information include the number of light-emitting portions 265 and the observation angles of the light-emitting portions 265 and the light-receiving portion 267.

When the scope 202 is connected to the light source 210, the scope-identification portion 263 reads out the scope information stored in the IC chip 261 and outputs the information to an effective-area defining portion 247. The effective-area defining portion 247 defines an effective area having a location and shape suitable for observing the observation target site X, on the basis of the scope information sent from the scope-identification portion 263. Depending on the shape of the defined effective area, the indication portion 51 shows the outline thereof or shows the inside and outside of the effective area in different colors.

The operation of the thus-configured fluorescence endoscope device 200 will now be described.

When an observation target site X inside the body cavity of a living body is observed using the fluorescence endoscope device 200 according to this embodiment, first, the scope-identification portion 263 identifies the scope information of any one of the scopes 202, 202A, and 202B connected to the light source 210 from the IC chip 261, and the scope information is inputted to the effective-area defining portion 247.

When the scope 202A, shown in FIG. 10, is connected, the effective-area defining portion 247 defines an effective area E such that it includes all the irradiation areas of the excitation light and reference light emitted from the light-emitting portions 265, on the basis of the scope information. In this case, the indication portion 51 shows the outline of the elliptical effective area E, as shown in FIG. 12.

On the other hand, when the scope 202B is connected, the effective-area defining portion 247 defines an effective area E such that a portion on a slightly peripheral side relative to the center of the image is to be observed, on the basis of the scope information. In this case, the indication portion 51 shows a ring-like effective area E excluding the central portion in a color different from that of the outside of the effective area E, as shown in FIG. 13.

As has been described above, with the fluorescence endoscope device 200 according to this embodiment, due to the effective-area defining portion 247 defining an effective area E based on the scope information, a practical and reliable effective area E can be defined for the scopes 202, 202A, and 202B each having different uses and specifications according to the observation object and the observation method.

Although the scope-identification portion 263 reads out the scope information from the IC chip 261 and outputs the information to the effective-area defining portion 247 in this embodiment, the scope information may be manually inputted to the effective-area defining portion 247 using an input device, such as a keyboard.

Furthermore, although the effective-area defining portion 247 defines an effective area E based on the scope information in this embodiment, it is also possible that, for example, an operator activates the effective-area defining portion 247 and manually defines an effective area E according to the observation object and the observation method.

### Third Embodiment

Next, a fluorescence endoscope device according to a third embodiment of the present invention will be described.

A fluorescence endoscope device 300 according to this embodiment differs from that according to the first embodiment in that, as shown in FIG. 14, an image-processing section 340 includes an information storage portion 371 that stores standard sample information obtained by observing a phantom (standard sample) Y and in that an effective-area defining portion 347 defines an effective area on the basis of the standard sample information.

Hereinbelow, portions having the same configurations as those of the fluorescence endoscope device 100 according to the first embodiment will be denoted by the same reference numerals, and descriptions thereof will be omitted.

A flat-plate-like object that emits fluorescence of uniform intensity substantially over the entire area is used as the phantom Y. For example, as shown in FIG. 15, this phantom Y has a fluorescence intensity indicated as a brightness of 2000 at the center of a corrected fluorescence image obtained when observed perpendicularly.

The information storage portion 371 can store an effective area defined by the effective-area defining portion 347 in the corrected fluorescence image of the phantom Y. For example, a region consisting of an area in which the variation in brightness is ±10% (an area having a fluorescence intensity indicated as a brightness from 1900 to 2100), an area in which the variation is from -10% to -20% (an area having a fluorescence intensity indicated as a brightness from 1800 to 1900), and an area in which the variation is from +10% to +20% (an area having a fluorescence intensity indicated as a brightness from 2100 to 2200), as shown in FIG. 16, is stored as an effective area E shown in FIG. 17.

The operation of the thus-configured fluorescence endoscope device 300 according to this embodiment will now be described.

When an observation target site X inside the body cavity of a living body is observed using the fluorescence endoscope device 300 according to this embodiment, once the image-correcting portion 43 generates a corrected fluorescence image of the observation target site X, the effective-area defining portion 347 reads out the effective area E of the phantom Y stored in the information storage portion 371, and the effective area E is defined in the corrected fluorescence image of the observation target site X.

Because the variation in brightness due to the influence of observation distance and observation angle clearly appears in the corrected fluorescence image of the phantom Y, which emits fluorescence of uniform intensity over the observation area, a precise effective area E can be defined in the corrected fluorescence image of the observation target site X, on the basis of the measured brightness in the corrected fluorescence image of the phantom Y. Thus, precise image information having little variation due to the influence of observation distance and observation angle can be obtained.

This embodiment may be modified as follows.

For example, as shown in FIG. 18, the fluorescence endoscope device 300 may include a selecting portion 373 that selects an arbitrary effective area E from an information storage portion 371 that stores effective areas E of a plurality of other phantoms having different shapes.

The other phantoms are phantoms formed of a fluorescent substance that emits fluorescence of uniform intensity over the entire area, and an example is a tubular phantom Z having a fluorescence intensity of a brightness of 2000, as shown in FIG. 19. When the scope 2 is inserted into the cavity of the phantom Z and when the inner wall is observed at a wide angle, a corrected fluorescence image in which the brightness is higher at the peripheral ring-like portion than at the center is generated, as shown in, for example, FIG. 20.

This corrected fluorescence image consists of an area including the center and having a fluorescence intensity indicated as a brightness of 1800 or less, an area therearound having a fluorescence intensity indicated as a brightness from 1800 to 2200, and an area therearound having a fluorescence intensity indicated as a brightness of 2200 or more. In this case, for example, as shown in FIG. 21, a ring-like region indicated as a brightness from 1800 to 2200, which is suitable for observing the inner wall of the esophagus or the large intestine at a wide angle, can be defined as an effective area E.

As has been described above, in this modification, by storing the effective areas E of a plurality of the phantoms Y having different shapes and fluorescence intensities in the information storage portion 371 in advance, and by activating the selecting portion 373 to select the effective area E of the phantom Y suitable for the observation object and the observation method to use it for the corrected fluorescence image of the observation target site X, a variety of observations can be precisely performed.

### Fourth Embodiment

Next, a fluorescence endoscope device according to a fourth embodiment of the present invention will be described.

A fluorescence endoscope device 400 according to this embodiment differs from that according to the first embodiment in that, as shown FIG. 22, an image-processing section 440 includes a condition determination portion 481 that determines the observation conditions and in that an effective-area defining portion 447 defines an effective area E according to an instruction from the condition determination portion 481.

Hereinbelow, portions having the same configurations as those of the fluorescence endoscope device 100 according to the first embodiment will be denoted by the same reference numerals, and descriptions thereof will be omitted.

The condition determination portion 481 defines a condition determination region of predetermined size in a white-light image generated by the image generating portion 41. The condition determination region is, for example, the area in a circle having a predetermined radius (a predetermined number of pixels) from the center of the image. Furthermore, the condition determination portion 461 determines whether or not the condition determination region contains a region having a brightness lower than or equal to a predetermined threshold.

For example, this condition determination portion 481 determines that the wall of the body cavity in the lumen of the esophagus, large intestine, or the like is observed at a wide angle, when a condition determination region C contains a region of pixels lower than the threshold, as shown in FIG. 23, and determines that observation is performed perpendicularly to the inner wall or the like, when the condition determination region C does not contain a region of pixels lower than the threshold, as shown in FIG. 24. The determination result determined by the condition determination portion 481 is outputted to the effective-area defining portion 447.

When the determination result that the observation is performed at a wide angle is inputted from the condition determination portion 481, the effective-area defining portion 447 defines a ring-like effective area E excluding the central portion of the corrected fluorescence image, as shown in FIG. 25. Furthermore, when the determination result that the observation is performed perpendicularly is inputted from the condition determination portion 481, the effective-area defining portion 447 defines a circular effective area E having a predetermined radius from the center of the corrected fluorescence image, as shown in FIG. 26.

With the thus-configured fluorescence endoscope device 400 according to this embodiment, by changing the shape of the effective area E on the basis of the determination result determined by the determination portion 481 corresponding to the observation condition, observation with a reliable effective area E can be performed even when the observation condition is changed.

Although the embodiments of the present invention have been described in detail with reference to the drawings, the specific configurations are not limited to those embodiments, but include design changes and the like. For example, the present invention may be applied not only to the above-described embodiments and modifications, but also to appropriate combinations of the above-described embodiments and modifications; it is not specifically limited.

### {Reference Signs List}

10: light source
21, 23: light-emitting portion
31: light-receiving portion
41: image generating portion (fluorescence-image acquisition portion and reference-image acquisition portion)
43: image-correcting portion (corrected-fluorescence-image generating portion)
45: region-extracting portion
47 : effective-area defining portion
51: indication portion
202, 202A, 202B: scope (endoscope scope)
H: high-brightness region
E: effective area

## Claims

1. A fluorescence endoscope device (100) comprising:
a light source (10) configured to irradiate a subject with excitation light and reference light;
a fluorescence-image acquisition portion (38, 41) configured to capture an image of fluorescence generated at the subject due to the irradiation with the excitation light from the light source (10) to obtain a fluorescence image;
a reference-image acquisition portion (39, 41) configured to capture an image of return light returning from the subject due to the irradiation with the reference light from the light source (10) to obtain a reference image;
a corrected-fluorescence-image generating portion (43) configured to correct the fluorescence image obtained by the fluorescence-image acquisition portion (38, 41) using the reference image obtained by the reference-image acquisition portion (39, 41) to generate a corrected fluorescence image;
an effective-area defining portion (47) configured to define an effective area having a brightness in a predetermined variation range in the corrected fluorescence image generated by the corrected-fluorescence-image generating portion (43);
a region-extracting portion (45) configured to extract a high-brightness region having a brightness higher than or equal to a predetermined threshold in the corrected fluorescence image; and
an indication portion (51) configured to show whether or not the high-brightness region extracted by the region-extracting portion (45) exists inside the effective area in an identifiable manner.

2. The fluorescence endoscope device (100) according to Claim 1, wherein the corrected-fluorescence-image generating portion (43) is configured to divide the fluorescence image by the reference image.

3. The fluorescence endoscope device (100) according to Claim 1 or 2, wherein the indication portion (51) is configured to show the effective area on the corrected fluorescence image such that the effective area is distinguishable from the other area.

4. The fluorescence endoscope device (100) according to any one of Claims 1 to 3, wherein the indication portion (51) is configured to show image information of the high-brightness region.

5. The fluorescence endoscope device (100) according to Claim 4, wherein the indication portion (51) is configured to show the image information of the high-brightness region in a vicinity thereof on the corrected fluorescence image.

6. The fluorescence endoscope device (100) according to any one of Claims 1 to 5, wherein the effective-area defining portion (47) is configured to define a predetermined area from a center of the corrected fluorescence image as the effective area.

7. The fluorescence endoscope device (100, 300) according to any one of Claims 1 to 6, wherein the effective-area defining portion (47, 347) is configured to define an effective area measured by using a standard sample that generates fluorescence of uniform intensity over an observation area as the subject as an effective area in a corrected fluorescence image of another subject.

8. The fluorescence endoscope device (100, 200) according to any one of Claims 1 to 7, further comprising an endoscope scope (2, 202) including, at a tip (2a) thereof, a light-emitting portion (265) configured to emit the excitation light and the reference light and a light-receiving portion (267) configured to receive the fluorescence and the return light, wherein the effective-area defining portion (47, 247) is configured to define an effective area on the basis of scope information about the light-emitting portion (265) and the light-receiving portion (267) of the endoscope scope (2, 202).

## Patentansprüche

1. Fluoreszenzendoskopvorrichtung (100), umfassend:
eine Lichtquelle (10), die dazu eingerichtet ist, ein Subjekt mit Anregungslicht und Referenzlicht zu bestrahlen;
einen Fluoreszenzbild-Erfassungsabschnitt (38, 41), der dazu eingerichtet ist, ein Bild der Fluoreszenz aufzunehmen, die bei dem Subjekt aufgrund der Bestrahlung mit dem Anregungslicht von der Lichtquelle (10) erzeugt wird, um ein Fluoreszenzbild zu erhalten;
einen Referenzbild-Erfassungsabschnitt (39, 41), der dazu eingerichtet ist, ein Bild des Rückkehrlichts aufzunehmen, das von dem Subjekt aufgrund der Bestrahlung mit dem Referenzlicht von der Lichtquelle (10) zurückkehrt, um ein Referenzbild zu erhalten;
einen Korrigiertes-Fluoreszenzbild-Erzeugungsabschnitt (43), der dazu eingerichtet ist, das durch den Fluoreszenzbild-Erfassungsabschnitt (38, 41) erhaltene Fluoreszenzbild unter Verwendung des durch den Referenzbild-Erfassungsabschnitt (39, 41) erhaltenen Referenzbilds zu korrigieren, um ein korrigiertes Fluoreszenzbild zu erzeugen;
einen Effektivbereich-Bestimmungsabschnitt (47), der dazu eingerichtet ist, einen effektiven Bereich zu bestimmen, der eine Helligkeit in einer vorbestimmten Variationsspanne in dem durch den Korrigiertes-Fluoreszenzbild-Erzeugungsabschnitt (43) erzeugten korrigierten Fluoreszenzbild aufweist;
einen Bereichsextraktionsabschnitt (45), der dazu eingerichtet ist, in dem korrigierten Fluoreszenzbild einen Hochhelligkeitsbereich zu extrahierten, der eine Helligkeit größer oder gleich einem vorbestimmten Schwellwert aufweist; und
einen Anzeigeabschnitt (51), der dazu eingerichtet ist, auf eine identifizierbare Weise anzuzeigen, ob der durch den Bereichsextraktionsabschnitt (45) extrahierte Hochhelligkeitsbereich innerhalb des effektiven Bereichs vorliegt oder nicht.

2. Fluoreszenzendoskopvorrichtung (100) nach Anspruch 1, wobei der Korrigiertes-Fluoreszenzbild-Erzeugungsabschnitt (43) dazu eingerichtet ist, das Fluoreszenzbild durch das Referenzbild zu teilen.

3. Fluoreszenzendoskopvorrichtung (100) nach Anspruch 1 oder 2, wobei der Anzeigeabschnitt (51) dazu eingerichtet ist, den effektiven Bereich auf dem korrigierten Fluoreszenzbild derart anzuzeigen, dass der effektive Bereich von dem anderen Bereich unterscheidbar ist.

4. Fluoreszenzendoskopvorrichtung (100) nach einem der Ansprüche 1 bis 3, wobei der Anzeigeabschnitt (51) dazu eingerichtet ist, Bildinformation des Hochhelligkeitsbereichs anzuzeigen.

5. Fluoreszenzendoskopvorrichtung (100) nach Anspruch 4, wobei der Anzeigeabschnitt (51) dazu eingerichtet ist, die Bildinformation des Hochhelligkeitsbereichs in einer Umgebung davon auf dem korrigierten Fluoreszenzbild anzuzeigen.

6. Fluoreszenzendoskopvorrichtung (100) nach einem der Ansprüche 1 bis 5, wobei der Effektivbereich-Bestimmungsabschnitt (47) dazu eingerichtet ist, einen vorbestimmten Bereich von einem Zentrum des korrigierten Fluoreszenzbilds als den effektiven Bereich zu bestimmen.

7. Fluoreszenzendoskopvorrichtung (100, 300) nach einem der Ansprüche 1 bis 6, wobei der Effektivbereich-Bestimmungsabschnitt (47, 347) dazu eingerichtet ist, einen effektiven Bereich zu bestimmen, der unter Verwendung einer Standardprobe gemessen wird, die Fluoreszenz gleichmäßiger Intensität über einen Beobachtungsbereich als das Subjekt als einen effektiven Bereich in einem korrigierten Fluoreszenzbild eines anderen Subjekts erzeugt.

8. Fluoreszenzendoskopvorrichtung (100, 200) nach einem der Ansprüche 1 bis 7, ferner umfassend einen Endoskopbereich (2, 202), der an einer Spitze (2a) davon einen Lichtemissionsabschnitt (265), der dazu eingerichtet ist, das Anregungslicht und das Referenzlicht zu emittieren, und einen Lichtempfangsabschnitt (267) aufweist, der dazu eingerichtet ist, das Fluoreszenz- und das Rückkehrlicht zu empfangen, wobei der Effektivbereich-Bestimmungsabschnitt (47, 247) dazu eingerichtet ist, einen effektiven Bereich auf Basis einer Bereichsinformation über den Lichtemissionsabschnitt (265) und den Lichtempfangsabschnitt (267) des Endoskopbereichs (2, 202) zu bestimmen.

## Revendications

1. Dispositif (100) d'endoscope à fluorescence comprenant :
une source de lumière (10) configurée pour irradier un sujet avec une lumière d'excitation et une lumière de référence ;
une partie (38, 41) d'acquisition d'image de fluorescence configurée pour capturer une image de fluorescence générée au niveau du sujet en raison de l'irradiation avec la lumière d'excitation provenant de la source de lumière (10) pour obtenir une image de fluorescence ;
une partie (39, 41) d'acquisition d'image de référence configurée pour capturer une image de la lumière de retour revenant depuis le sujet en raison de l'irradiation avec la lumière de référence provenant de la source de lumière (10) pour obtenir une image de référence ;
une partie (43) de génération d'image de fluorescence corrigée configurée pour corriger l'image de fluorescence obtenue par la partie (38, 41) d'acquisition d'image de fluorescence en utilisant l'image de référence obtenue par la partie (39, 41) d'acquisition d'image de référence pour générer une image de fluorescence corrigée ;
une partie (47) de définition de zone effective configurée pour définir une zone effective présentant une luminosité dans une plage de variation prédéterminée dans l'image de fluorescence corrigée générée par la partie (43) de génération d'image de fluorescence corrigée ;
une partie (45) d'extraction de région configurée pour extraire une région à luminosité élevée présentant une luminosité supérieure ou égale à un seuil prédéterminé dans l'image de fluorescence corrigée ; et
une partie (51) d'indication configurée pour montrer si oui ou non la région à luminosité élevée extraite par la partie (45) d'extraction de région se trouve à l'intérieur de la zone effective de manière identifiable.

2. Dispositif (100) d'endoscope à fluorescence selon la revendication 1, dans lequel la partie (43) de génération d'image de fluorescence corrigée est configurée pour diviser l'image de fluorescence par l'image de référence.

3. Dispositif (100) d'endoscope à fluorescence selon la revendication 1 ou 2, dans lequel la partie (51) d'indication est configurée pour montrer la zone effective sur l'image de fluorescence corrigée d'une manière telle que la zone effective est distinguable de l'autre zone.

4. Dispositif (100) d'endoscope à fluorescence selon l'une quelconque des revendications 1 à 3, dans lequel la partie (51) d'indication est configurée pour montrer une information d'image de la région à luminosité élevée.

5. Dispositif (100) d'endoscope à fluorescence selon la revendication 4, dans lequel la partie (51) d'indication est configurée pour montrer l'information d'image de la région à luminosité élevée au voisinage de celle-ci sur l'image de fluorescence corrigée.

6. Dispositif (100) d'endoscope à fluorescence selon l'une quelconque des revendications 1 à 5, dans lequel la partie (47) de définition de zone effective est configurée pour définir une zone prédéterminée à partir d'un centre de l'image de fluorescence corrigée en tant que zone effective.

7. Dispositif (100, 300) d'endoscope à fluorescence selon l'une quelconque des revendications 1 à 6, dans lequel la partie (47, 347) de définition de zone effective est configurée pour définir une zone effective mesurée en utilisant un échantillon standard qui génère une fluorescence d'une intensité uniforme sur une zone d'observation comme le sujet en tant que zone effective dans une image de fluorescence corrigée d'un autre sujet.

8. Dispositif (100, 200) d'endoscope à fluorescence selon l'une quelconque des revendications 1 à 7, comprenant en outre un scope d'endoscope (2, 202) comprenant, au niveau d'une pointe (2a) de celui-ci, une partie (265) d'émission de lumière configurée pour émettre la lumière d'excitation et la lumière de référence et une partie (267) de réception de lumière configurée pour recevoir la fluorescence et la lumière de retour, dans lequel la partie (47, 247) de définition de zone effective est configurée pour définir une zone effective sur la base d'une information de scope concernant la partie (265) d'émission de lumière et la partie (267) de réception de lumière du scope d'endoscope (2, 202).
